(19) Europäisches Patentamt
European Patent Office
Office européen des brevets

(11) EP 3 376 076 A1

(12) **EUROPEAN PATENT APPLICATION**

(43) Date of publication:
**19.09.2018 Bulletin 2018/38**

(21) Application number: **17204605.4**

(22) Date of filing: **30.11.2017**

(51) Int Cl.:
*F16J 1/00* (2006.01) *A61M 5/00* (2006.01)
*B29C 39/00* (2006.01) *B32B 25/00* (2006.01)
*F16J 15/3204* (2016.01) *F16J 15/3284* (2016.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **14.03.2017 JP 2017048675**

(71) Applicant: **Sumitomo Rubber Industries, Ltd.**
**Kobe-shi, Hyogo-ken (JP)**

(72) Inventor: **MINAGAWA, Yasuhisa**
**Kobe-shi, Hyogo 651-0072 (JP)**

(74) Representative: **Manitz Finsterwald Patentanwälte PartmbB**
**Martin-Greif-Strasse 1**
**80336 München (DE)**

(54) **GASKET**

(57) The present invention provides a gasket (2) excellent in properties such as sliding properties and resistance to liquid leakage. The present invention relates to a gasket including a gasket base material (1) whose surface is at least partially provided with immobilized polymer chains (21), the gasket having a sliding surface (14) provided with multiple annular projections (14a, 14b, 14c), the annular projections including a first projection (14a) nearest to the top surface (12) of the gasket, the first projection having a surface roughness Ra of 1.0 or less.

Fig. 2

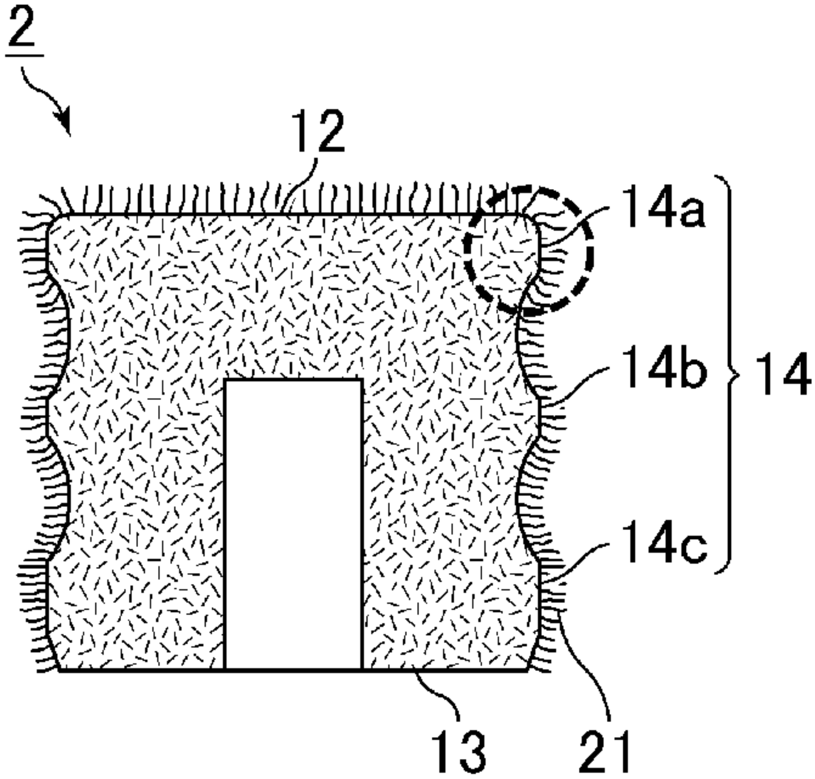




Printed by Jouve, 75001 PARIS (FR)

## Description

TECHNICAL FIELD

The present invention relates to a gasket.

BACKGROUND ART

In view of the importance of resistance to liquid leakage, elastic bodies such as rubber are used in parts which slide while maintaining a seal, e.g., a gasket which is integrated with a plunger of a syringe to form a seal between the plunger and the barrel. Unfortunately, such elastic bodies have a slight problem with sliding properties (see Patent Literature 1). To address this problem, a sliding property-improving agent, for example silicone oil, is applied to the sliding surface; however, a concern has been raised over the potential adverse effects of silicone oil on recently marketed bio-preparations. On the other hand, gaskets not coated with a sliding property-improving agent have inferior sliding properties and therefore do not allow plungers to be smoothly pushed but cause them to pulsate during administration. This results in problems such as inaccurate injection amounts and infliction of pain on patients.

To satisfy the conflicting requirements, i.e., resistance to liquid leakage and sliding properties, a method of coating surfaces with a self-lubricating PTFE film has been proposed (see Patent Literature 2). Unfortunately, such PTFE films are generally expensive and thus increase the production cost of processed products, limiting the range of application of the method. Also, products coated with PTFE films might be unreliable when they are used in applications where sliding or similar movement is repeated and durability is therefore required. Furthermore, since PTFE is vulnerable to radiation, PTFE-coated products unfortunately cannot be sterilized by radiation.

CITATION LIST

PATENT LITERATURE

Patent Literature 1: JP 2004-298220 A
Patent Literature 2: JP 2010-142573 A

SUMMARY OF INVENTION

TECHNICAL PROBLEM

The present invention aims to solve the above problems and provide a gasket excellent in properties such as sliding properties and resistance to liquid leakage.

SOLUTION TO PROBLEM

The present invention relates a gasket, including a gasket base material whose surface is at least partially provided with immobilized polymer chains, the gasket having a sliding surface provided with multiple annular projections, the annular projections including a first projection nearest to a top surface of the gasket, the first projection having a surface roughness Ra of 1.0 or less.

Preferably, the first projection has a surface roughness Ra of 0.8 or less.

Preferably, the first projection has a surface roughness Ra of 0.6 or less.

Preferably, the gasket base material has a surface roughness Ra of 1.0 or less.

Preferably, the gasket base material has a surface roughness Ra of 0.8 or less.

Preferably, the gasket base material has a surface roughness Ra of 0.6 or less.

Preferably, the polymer chains are immobilized by a surface modification method I including: Step 1 of forming polymerization initiation points A on the surface of the gasket base material; and Step 2 of radically polymerizing a monomer starting from the polymerization initiation points A to grow polymer chains.

Preferably, the surface modification method I includes: Step 3 of extending the polymer chains grown in Step 2 with the same type or a different type of polymer chain; or Step 3' of attaching a silane compound to surfaces of the polymer chains grown in Step 2, followed by reaction with a perfluoroether group-containing silane compound to grow functional polymer chains.

Preferably, Step 1 includes adsorbing a photopolymerization initiator A onto the surface of the gasket base

material, optionally followed by irradiation with LED light having a wavelength of 300 to 450 nm, to form polymerization initiation points A from the photopolymerization initiator A on the surface.

Preferably, Step 2 includes radically polymerizing a monomer starting from the polymerization initiation points A by irradiation with LED light having a wavelength of 300 to 450 nm to grow polymer chains.

Preferably, the polymer chains are immobilized by a surface modification method II including Step I of radically polymerizing a monomer in the presence of a photopolymerization initiator A on the surface of the gasket base material to grow polymer chains.

Preferably, the surface modification method II includes: Step II of extending the polymer chains grown in Step I with the same type or a different type of polymer chain; or Step II' of attaching a silane compound to surfaces of the polymer chains grown in Step I, followed by reaction with a perfluoroether group-containing silane compound to grow functional polymer chains.

Preferably, Step I includes radically polymerizing a monomer by irradiation with LED light having a wavelength of 300 to 450 nm to grow polymer chains.

Preferably, the polymer chains have a length of 500 to 5,000 nm.

## ADVANTAGEOUS EFFECTS OF INVENTION

The gasket of the present invention includes a gasket base material whose surface is at least partially provided with immobilized polymer chains, the gasket having a sliding surface provided with multiple annular projections, the annular projections including a first projection nearest to the top surface of the gasket, the first projection having a surface roughness Ra of 1.0 or less. Thus, the present invention provides a gasket excellent in properties such as sliding properties and resistance to liquid leakage without applying any sliding property-improving agent that can adversely affect chemical liquids, e.g., silicone oil, to the sliding surface.

## BRIEF DESCRIPTION OF DRAWINGS

Fig. 1 is an exemplary longitudinal cross-sectional view of a gasket base material on which polymer chains are to be immobilized.

Fig. 2 is an exemplary longitudinal cross-sectional view of a gasket in which polymer chains are immobilized on the surface of a gasket base material.

Fig. 3 is an exemplary partial enlarged view of a first projection of the gasket shown in Fig. 2.

## DESCRIPTION OF EMBODIMENTS

The gasket of the present invention includes a gasket base material whose surface is at least partially provided with immobilized polymer chains. Further, the gasket has a sliding surface provided with multiple annular projections. Further, the multiple annular projections of the gasket include a first projection nearest to the top surface, and the first projection has a surface roughness Ra of 1.0 or less. Due to the polymer chains immobilized on the surface of the base material and the controlled surface roughness Ra of 1.0 or less of at least the first projection located nearest to the top surface, high sliding properties and high resistance to liquid leakage can be simultaneously achieved.

An exemplary preferred embodiment of the present invention will be described below referring to drawings.

Fig. 1 is an exemplary longitudinal cross-sectional view (cross-sectional view in the sliding direction (longitudinal cross-section)) of a base material 1 (gasket base material 1) on which polymer chains are to be immobilized. Fig. 2 is an exemplary longitudinal cross-sectional view of a gasket 2 of the present invention in which polymer chains 21 are immobilized on the surface of the gasket base material 1 shown in Fig. 1. Fig. 3 is an exemplary partial enlarged view (area enclosed by the circle) of a first projection 14a of the gasket 2 shown in Fig. 2.

The gasket 2 can be used in, for example, a syringe that includes a barrel into which liquid is injected, a plunger for pushing the injected liquid out of the barrel, and a gasket attached to the tip of the plunger.

The gasket 2 in Fig. 2 is prepared by immobilizing polymer chains on at least a part of the sliding surface of the gasket base material 1 shown in Fig. 1. In the gasket 2 including the straight cylindrical gasket base material 1 on which polymer chains 21 are immobilized, the circumference of a top surface 12 on the liquid-contact side and the circumference of a bottom surface 13 to be connected to the tip of a plunger are integrated with a sliding portion 14 (cylindrical portion) extending in the height direction (sliding direction).

With regard to the gasket base material 1 or gasket 2, the outer periphery of the sliding portion 14 includes three annular projections that make sliding contact with the inner periphery of the peripheral cylindrical portion of the barrel; specifically, a first projection 14a at a position nearest to the top surface 12 (first projection 14a nearest to the

top surface), a bottom projection 14c at a position farthest from the top surface 12 (bottom projection 14c nearest to the bottom surface), and an intermediate projection 14b at a position between the projections 14a and 14c. In the gasket base material shown in Fig. 1, the top surface 12 is integrated with the first projection 14a.

Although Figs. 1 and 2 show an embodiment having three annular projections, there may be any number, but at least two, of annular projections. Although the embodiment has one intermediate projection 14b, any projection between the first projection and the bottom projection corresponds to an intermediate projection, and there may be multiple intermediate projections.

In order to simultaneously achieve sliding properties and resistance to liquid leakage, the gasket 2 preferably has three or more annular projections. The top surface 12 on the liquid-contact side, the bottom surface 13 to be connected to the tip of a plunger, the first projection 14a, the intermediate projection 14b, the bottom projection 14c, and the sliding portion 14 in the straight cylindrical gasket base material 1 or gasket 2 may each have any shape.

The gasket 2 in Fig. 2 or Fig. 3 (the partial enlarged view of the first projection 14a) is prepared by immobilizing polymer chains 21 on at least a part of the surface of the gasket base material 1. The figures show an example in which polymer chains 21 are immobilized on the top surface 12 and the entire sliding portion 14 (cylindrical portion) including annular projections (first projection 14a, intermediate projection 14b, and bottom projection 14c).

In order to simultaneously achieve sliding properties and resistance to liquid leakage, in the gasket 2 (after the immobilization of polymer chains), the first projection 14a provided with polymer chains 21 has a surface roughness Ra of 1.0 or less, preferably 0.8 or less, more preferably 0.6 or less. The lower limit is not particularly critical, and a smaller Ra is better.

The surface roughness Ra herein refers to a center-line surface roughness Ra defined in JIS B0601-2001.

In order to simultaneously achieve sliding properties and resistance to liquid leakage, the first projection 14a in the gasket base material 1 (before the immobilization of polymer chains) preferably has a surface roughness Ra of 1.0 or less, more preferably 0.8 or less, still more preferably 0.6 or less. The lower limit is not particularly critical, and a smaller Ra is better.

The surface roughness Ra of the gasket base material 1 or the gasket 2 in which polymer chains 21 are immobilized on the gasket base material 1 can be controlled, for example, by varying the surface roughness of the forming mold, specifically by varying the particle size of the abrasive used in the final finishing step in the production of the mold. Examples of the abrasive include abrasive grains made of diamond, alumina, silicon carbide, cubic boron nitride, boron carbide, zirconium oxide, manganese oxide, colloidal silica, or other materials. Suitable examples include those of #46 to #100 defined in JIS R6001-1998.

The material of the forming mold may be a known material, such as carbon steel or precipitation hardening stainless steel. The forming mold can be produced by cutting methods, such as by cutting with a cemented carbide tool, coated cemented carbide, sintered cBN, or other tools, followed by polishing and finishing.

As described above, the gasket of the present invention is prepared by immobilizing polymer chains on at least a part of the surface of a gasket base material. The gasket has a sliding surface provided with multiple annular projections. Any polymer chain can be used, including polymer chains formed by polymerization of conventionally known monomers. The polymer chains may be immobilized by any method, including known methods such as the "grafting from" method in which graft polymerization of monomers is initiated from the surface and the "grafting to (on)" method in which polymer chains are reacted with and immobilized on the surface.

Such a gasket of the present invention can be produced, for example, by subjecting a gasket base material having a sliding surface provided with multiple annular projections to a surface modification method as described below.

The gasket of the present invention can be produced by immobilizing polymer chains using a surface modification method I that includes: Step 1 of forming polymerization initiation points A on the surface of a gasket base material; and Step 2 of radically polymerizing a monomer starting from the polymerization initiation points A to grow polymer chains.

Step 1 includes forming polymerization initiation points A on the surface of a vulcanized rubber or a formed thermoplastic elastomer (gasket base material).

The vulcanized rubber or the thermoplastic elastomer may suitably be one containing a carbon atom adjacent to a double bond (i.e., allylic carbon atom).

Examples of the rubber include diene rubbers such as styrene-butadiene rubber, polybutadiene rubber, polyisoprene rubber, natural rubber, and deproteinized natural rubber; butyl rubber and halogenated butyl rubber which have a degree of unsaturation of a few percent of isoprene units; and silicone rubber. In the case of butyl rubber or halogenated butyl rubber, it is preferably a rubber crosslinked by triazine because the amount of matter extracted from the vulcanized rubber is reduced. In such a case, the rubber may contain an acid acceptor. Suitable examples of the acid acceptor include hydrotalcite and magnesium carbonate.

In cases where other rubbers are used, sulfur vulcanization is preferably performed. In such cases, compounding ingredients commonly used in sulfur vulcanization may be added, such as vulcanization accelerators, zinc oxide, fillers, and silane coupling agents. Suitable fillers include carbon black, silica, clay, talc, and calcium carbonate.

The vulcanization conditions for the rubber may be appropriately selected. The rubber is preferably vulcanized

at a temperature of 150°C or higher, more preferably 170°C or higher, still more preferably 175°C or higher.

Examples of the thermoplastic elastomer include polymer compounds that have rubber elasticity at room temperature owing to aggregates of plastic components (hard segments) serving as crosslinking points (e.g., thermoplastic elastomers (TPE) such as styrene-butadienestyrene copolymers); and polymer compounds having rubber elasticity produced by mixing thermoplastic and rubber components and dynamically crosslinking the mixture by a crosslinking agent (e.g., thermoplastic elastomers (TPV) such as polymer alloys containing combinations of styrenic block copolymers or olefinic resins with crosslinked rubber components).

Other suitable thermoplastic elastomers include nylon, polyester, polyurethane, polypropylene, fluoroelastomers such as PTEF, and dynamically crosslinked thermoplastic elastomers thereof. Preferred among dynamically crosslinked thermoplastic elastomers are those produced by dynamically crosslinking halogenated butyl rubber in thermoplastic elastomer. In this case, the thermoplastic elastomer is preferably, for example, nylon, polyurethane, polypropylene, styrene-isobutylene-styrene block copolymer (SIBS).

The polymerization initiation points A may be formed, for example, by adsorbing a photopolymerization initiator A onto the surface of the gasket base material. Examples of the photopolymerization initiator A include carbonyl compounds, organic sulfur compounds such as tetraethylthiuram disulfide, persulfides, redox compounds, azo compounds, diazo compounds, halogen compounds, and photoreductive pigments. Carbonyl compounds are preferred among these.

The carbonyl compound used as a photopolymerization initiator A is preferably benzophenone or its derivative, and may suitably be a benzophenone compound represented by the following formula:

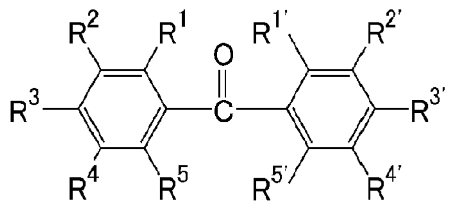

wherein $R^1$ to $R^5$ and $R^{1'}$ to $R^{5'}$ are the same as or different from one another and each represent a hydrogen atom, an alkyl group, a halogen (fluorine, chlorine, bromine, or iodine), a hydroxy group, a primary to tertiary amino group, a mercapto group, or a hydrocarbon group optionally containing an oxygen atom, a nitrogen atom, or a sulfur atom; and any adjacent two of $R^1$ to $R^5$ and $R^{1'}$ to $R^{5'}$ may be joined to each other to form a cyclic structure together with the carbon atoms to which they are attached.

Specific examples of the benzophenone compound include benzophenone, xanthone, 9-fluorenone, 2,4-dichlorobenzophenone, methyl o-benzoylbenzoate, 4,4'-bis(dimethylamino)benzophenone, and 4,4'-bis(diethylamino)benzophenone. Among these, benzophenone, xanthone, and 9-fluorenone are particularly preferred because they allow polymer brushes to be well formed.

Other suitable examples of the benzophenone compound include fluorobenzophenone compounds, such as 2,3,4,5,6-pentafluorobenzophenone and decafluorobenzophenone respectively represented by the following formulas.

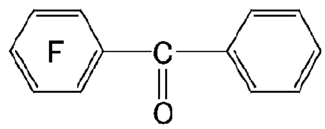

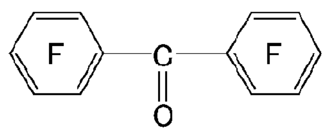

Thioxanthone compounds can also be suitably used as the photopolymerization initiator A because they provide a high polymerization rate and also can readily be adsorbed onto and/or reacted with rubber or the like. For example, compounds represented by the following formula can be suitably used.

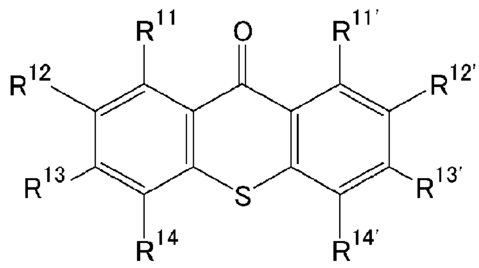

In the formula, $R^{11}$ to $R^{14}$ and $R^{11'}$ to $R^{14'}$ are the same as or different from one another and each represent a hydrogen atom, a halogen atom, an alkyl group, a cyclic alkyl group, an aryl group, an alkenyl group, an alkoxy group, or an aryloxy group.

Examples of thioxanthone compounds represented by the above formula include thioxanthone, 2-isopropylthioxanthone, 4-isopropylthioxanthone, 2,3-diethylthioxanthone, 2,4-diethylthioxanthone, 2,4-dichlorothioxanthone, 2-methoxythioxanthone, 1-chloro-4-propoxythioxanthone, 2-cyclohexylthioxanthone, 4-cyclohexylthioxanthone, 2-vinylthioxanthone, 2,4-divinylthioxanthone, 2,4-diphenylthioxanthone, 2-butenyl-4-phenylthioxanthone, 2-methoxythioxanthone, and 2-p-octyloxyphenyl-4-ethylthioxanthone. Preferred among these are those which are substituted at one or two, particularly two, of $R^{11}$ to $R^{14}$ and $R^{11'}$ to $R^{14'}$ with alkyl groups. More preferred is 2,4-diethylthioxanthone.

The photopolymerization initiator A such as a benzophenone or thioxanthone compound can be adsorbed onto the surface of the gasket base material by known methods. For example, in the case of a benzophenone or thioxanthone compound, the benzophenone or thioxanthone compound is dissolved in an organic solvent to prepare a solution, and a surface portion of the gasket base material to be modified is treated with this solution so that the compound is adsorbed onto the surface, optionally followed by evaporating off the organic solvent by drying, to form polymerization initiation points. The surface may be treated by any method that allows the solution of the benzophenone or thioxanthone compound to be brought into contact with the surface of the gasket base material. Suitable examples of the surface treatment method include application or spraying of the benzophenone or thioxanthone compound solution; and immersion into the solution. In the case where only a part of the surface needs to be modified, it is sufficient to adsorb the photopolymerization initiator A only onto the desired part of the surface. In this case, for example, application or spraying of the solution is suitable. Examples of the solvent include methanol, ethanol, acetone, benzene, toluene, methyl ethyl ketone, ethyl acetate, and THF. Acetone is preferred because it does not swell the gasket base material and it rapidly dries and evaporates.

After the portion on which polymer chains are to be immobilized is surface treated with the benzophenone or thioxanthone compound solution so that the photopolymerization initiator A is adsorbed onto the portion, the surface of the gasket base material is preferably further irradiated with light so that the polymerization initiator A is chemically bonded to the surface. For example, the benzophenone or thioxanthone compound may be immobilized on the surface by irradiation with ultraviolet light having a wavelength of 300 to 450 nm, preferably 300 to 400 nm, more preferably 350 to 400 nm. During Step 1 and the immobilization process, a hydrogen atom is abstracted from the rubber surface and a carbon atom on the rubber surface is then covalently bonded to the carbon atom in C=O of benzophenone, while the abstracted hydrogen atom is bonded to the oxygen atom in C=O to form C-O-H, as shown in the scheme below. Moreover, since such a hydrogen abstraction reaction selectively occurs on allylic hydrogen atoms in the gasket base material, the rubber preferably contains a butadiene or isoprene unit that contains an allylic hydrogen atom.

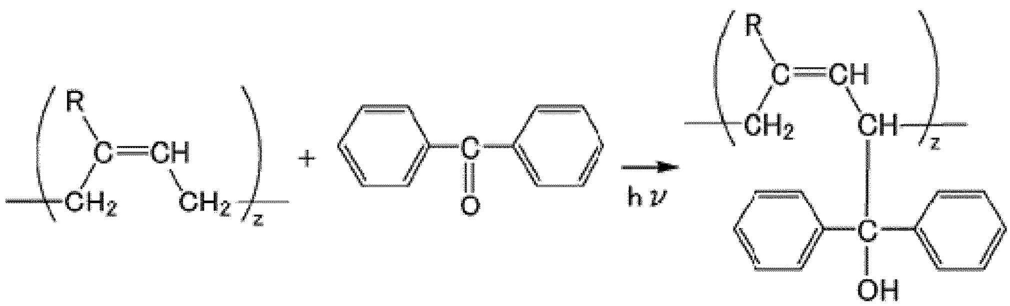


R: a hydrogen atom or a C1-C4 alkyl group

In particular, the polymerization initiation points A are preferably formed by treating the surface of the gasket base material with the photopolymerization initiator A so that the photopolymerization initiator A is adsorbed onto the surface, and then irradiating the treated surface with LED light having a wavelength of 300 to 450 nm. Particularly

preferably, after the surface of the gasket base material is treated with the benzophenone or thioxanthone compound solution so that the photopolymerization initiator A is adsorbed, the treated surface is further irradiated with LED light having a wavelength of 300 to 450 nm so that the adsorbed photopolymerization initiator A is chemically bonded to the surface. Since light having a wavelength of less than 300 nm may break and damage the molecules in the gasket base material, light having a wavelength of 300 nm or more is preferably used. Light having a wavelength of 355 nm or more is more preferred in that such light causes only very small damage to the gasket base material. Also, since light having a wavelength of more than 450 nm is less likely to activate the polymerization initiator and thus less likely to allow the polymerization reaction to proceed, light having a wavelength of 450 nm or less is preferred. Light having a wavelength of 400 nm or less is more preferred for greater activation of the polymerization initiator. LED light having a wavelength of 355 to 380 nm is particularly suitable. Although LED light is suitable in that the wavelength range of LED light is narrow so that no wavelengths other than the center wavelength are emitted, mercury lamps or other light sources can also produce similar effects to those of LED light by using a filter to block light with wavelengths less than 300 nm.

Step 2 includes radically polymerizing a monomer starting from the polymerization initiation points A to grow polymer chains.

Non-limiting examples of the monomer include hydroxyalkyl (meth)acrylates such as hydroxyethyl (meth)acrylate and hydroxybutyl (meth)acrylate, (meth)acrylic acid, dimethyl (meth)acrylamide, diethyl (meth)acrylamide, isopropyl (meth)acrylamide, hydroxyethyl (meth)acrylamide, methoxymethyl (meth)acrylamide, (meth)acrylamide, methoxymethyl (meth)acrylate, and (meth)acrylonitrile. These monomers may be used alone, or two or more of these may be used in combination. In view of cost efficiency, the monomer used in Step 2 is preferably (meth)acrylic acid, a hydroxyalkyl (meth)acrylate, dimethyl (meth)acrylamide, diethyl (meth)acrylamide, isopropyl (meth)acrylamide, hydroxyethyl (meth)acrylamide, methoxymethyl (meth)acrylamide, (meth)acrylamide, or methoxymethyl (meth)acrylate, more preferably (meth)acrylic acid or (meth)acrylamide, still more preferably acrylic acid or acrylamide, among others.

The monomer may suitably be a fluorine-containing monomer.

Examples of the fluorine-containing monomer include fluorine-containing (meth)acrylic-modified organosilicon compounds and cyclic siloxanes. The fluorine-containing monomer preferably contains a perfluoropolyether group in order to better achieve the effects of the present invention.

The fluorine-containing monomer may suitably be, for example, a fluorine-containing (meth)acrylic-modified organosilicon compound produced by an addition reaction of (B) an unsaturated monocarboxylic acid containing a (meth)acrylic group with (A) a fluorine-containing epoxy-modified organosilicon compound represented by the following formula (1) :

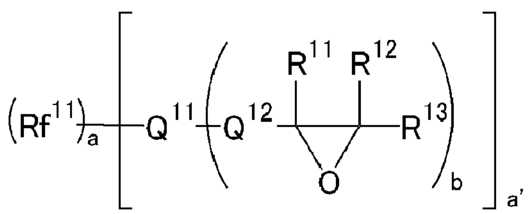

(1)

wherein $Rf^{11}$ represents a monovalent or divalent group having a molecular weight of 100 to 40,000 and containing a fluoroalkyl structure or a fluoropolyether structure; $Q^{11}$ represents an (a+b)-valent linking group containing at least (a+b) silicon atoms and having a siloxane structure, an unsubstituted or halogen-substituted silalkylene structure, a silarylene structure, or a combination of two or more thereof, and $Q^{11}$ may form a cyclic structure; $Q^{12}$ represents a C1-20 divalent hydrocarbon group which may form a cyclic structure and may be interrupted by an ether linkage (-O-) or an ester linkage (-COO-) ; $R^{11}$ to $R^{13}$ each independently represent a hydrogen atom or a C1-10 monovalent hydrocarbon group, provided that a part or all of the hydrogen atoms of $R^{11}$ to $R^{13}$ may be substituted with halogen atoms, and $R^{11}$ and $R^{12}$ may be joined to each other to form a ring together with the carbon atoms to which they are attached; when $Rf^{11}$ is a monovalent group, a' and a represent 1 and an integer of 1 to 6, respectively, and when $Rf^{11}$ is a divalent group, a and a' represent 1 and 2, respectively; and b represents an integer of 1 to 20.

With regard to the fluorine-containing epoxy-modified organosilicon compound (A), specific examples of $Q^{11}$ in formula (1) include groups having the structures represented by the following formulas.

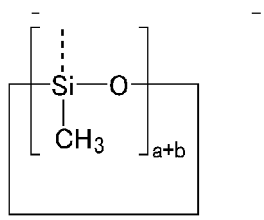

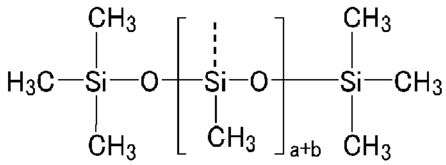

In the formulas, a and b are as defined above and are each preferably an integer of 1 to 4. Moreover, (a+b) is preferably an integer of 3 to 5. The unit repeated a times and the unit repeated b times are randomly arranged. The bond represented by the broken line in each of the units repeated a times and b times is attached to $Rf^{11}$ or the group represented by the following formula:

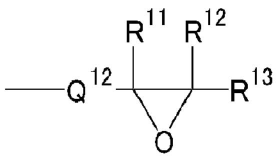

wherein $Q^{12}$ and $R^{11}$ to $R^{13}$ are as defined above.

The divalent hydrocarbon group for $Q^{12}$ in formula (1) preferably has 2 to 15 carbon atoms. Specific examples of the structure of $Q^{12}$ include $-CH_2CH_2-$, $-CH_2CH(CH_3)-$, and $-CH_2CH_2CH_2OCH_2-$.

The monovalent hydrocarbon group for $R^{11}$ to $R^{13}$ preferably has 1 to 8 carbon atoms. Specific examples of $R^{11}$ to $R^{13}$ include a hydrogen atom, alkyl groups such as methyl, ethyl, and propyl groups, and cycloalkyl groups such as cyclopentyl and cyclohexyl groups.

Examples of the group containing a combination of $R^{11}$ to $R^{13}$ and $Q^{12}$ represented by the above formula include the following groups.

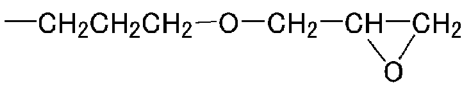

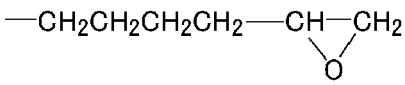

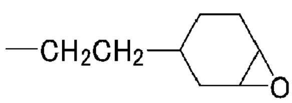

$Rf^{11}$ in formula (1) preferably has a molecular weight of 500 to 20,000. Moreover, $Rf^{11}$ may suitably contain 1 to 500, preferably 2 to 400, more preferably 4 to 200 repeating units of the formula: $-C_iF_{2i}O-$ where i in each unit independently represents an integer of 1 to 6. In the present invention, the term "molecular weight" refers to a number average molecular weight calculated from the ratio between the chain end structure and the backbone structure as determined by $^1$H-NMR and $^{19}$F-NMR.

Examples of $Rf^{11}$ in formula (1) include groups represented by the following formula (3):

$$-\left[Q^{13}-Rf'^{11}-Q^{13}-T\right]_v-Q_f^{11}-Rf'^{11}-Q^{13}- \quad (3)$$

wherein Rf'$^{11}$ represents a divalent perfluoropolyether group having a molecular weight of 300 to 30,000 which may be internally branched; $Q^{13}$ represents a divalent organic group which may contain an oxygen atom, a nitrogen atom, a fluorine atom, or a silicon atom, and may contain a cyclic structure or an unsaturated bond; $Q_f^{11}$ represents $Q^{13}$ or a fluorine atom; T represents a linking group represented by the following formula (4):

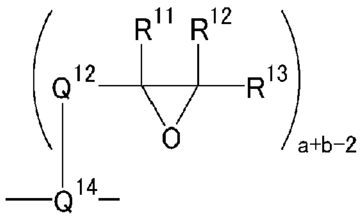

(4)

wherein $R^{11}$ to $R^{13}$, $Q^{12}$, a, and b are as defined in formula (1), and $Q^{14}$ represents an (a+b)-valent linking group containing at least (a+b) silicon atoms and having a siloxane structure, an unsubstituted or halogen-substituted silalkylene structure, a silarylene structure, or a combination of two or more thereof; and v represents an integer of 0 to 5, provided that v is 0 when $Q_f^{11}$ is a fluorine atom.

Rf'$^{11}$ in formula (3) preferably has a molecular weight of 500 to 20,000. Specific examples of Rf'$^{11}$ include divalent perfluoropolyether groups represented by the following formulas:

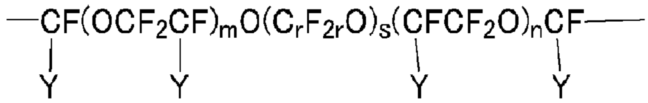

wherein each Y independently represents a fluorine atom or $CF_3$ group; r represents an integer of 2 to 6; m and n each represent an integer of 0 to 200, preferably 0 to 100, provided that (m+n) is an integer of 2 to 200, preferably 3 to 150; s represents an integer of 0 to 6; and the repeating units may be randomly linked, and

**$-C_jF_{2j}(OCF_2CF_2CF_2)_kOC_jF_{2j}-$**

wherein j represents an integer of 1 to 3, and k represents an integer of 1 to 200, preferably 1 to 60.

Examples of $Q^{13}$ in formula (3) include the following groups:

**$-CH_2CH_2-$**

**$-CH_2CH_2CH_2-$**

**$-CH_2OCH_2CH_2CH_2-$**

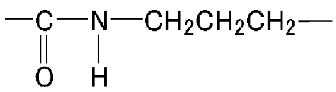

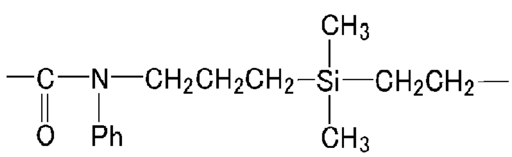

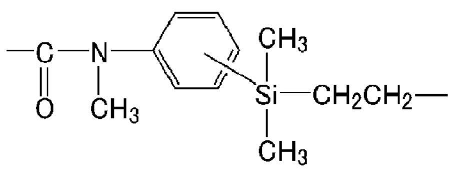

wherein Ph represents a phenyl group.

In formula (1), when $Rf^{11}$ is a monovalent group, a is preferably an integer of 1 to 3; b is preferably an integer of 1 to 6; and (a+b) is preferably an integer of 3 to 6.

Specific examples of $Rf^{11}$ in formula (1) include the following groups:

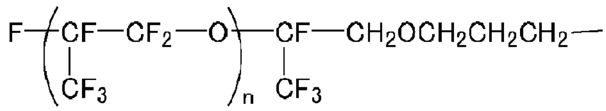

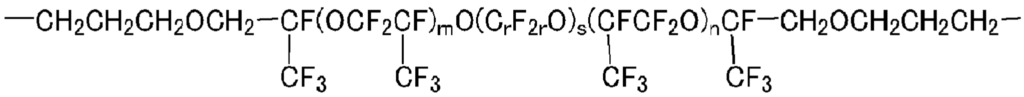

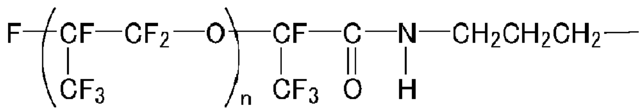

wherein m, n, r, and s are as defined above.

Specific examples of the fluorine-containing epoxy-modified organosilicon compound (A) include the following compounds:

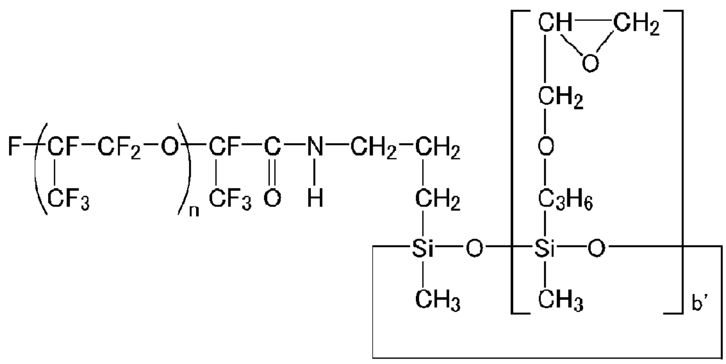

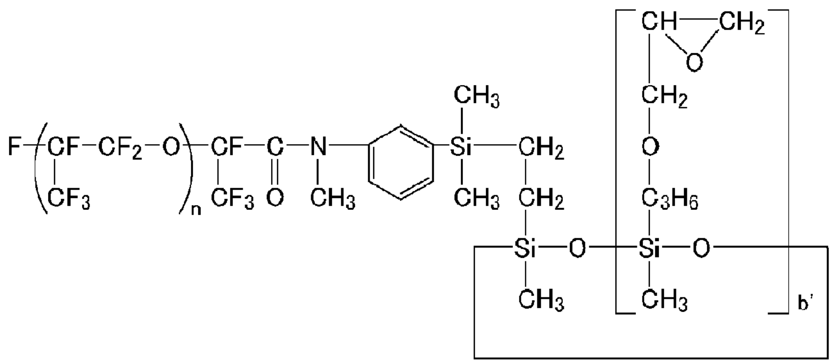

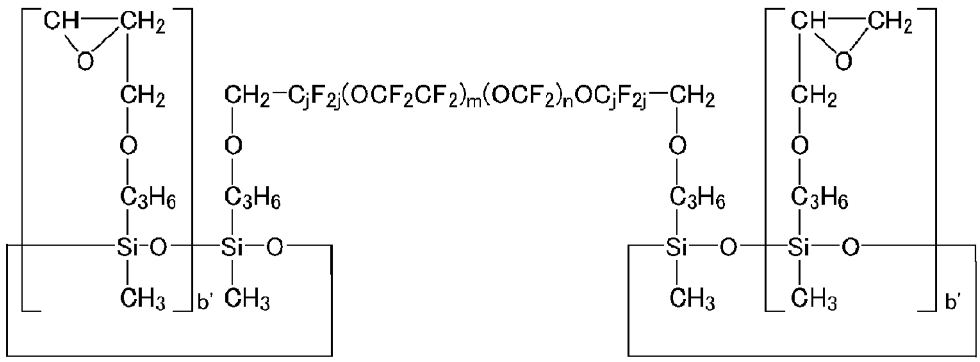

wherein j, m, and n are as defined above, and b' is an integer of 1 to 8.

These fluorine-containing epoxy-modified organosilicon compounds may be used alone, or two or more of these may be used in combination.

The unsaturated monocarboxylic acid (B) containing a (meth)acrylic group may suitably be acrylic acid or methacrylic acid and may also be one in which a part of the hydrogen atoms is halogenated with a halogen atom (e.g. chlorine, fluorine), such as 2-chloroacrylic acid, 2-(trifluoromethyl)acrylic acid, or 2,3,3-trifluoroacrylic acid. The carboxylic acids may optionally be protected by an allyl group, a silyl group, or other groups. The unsaturated monocarboxylic acids may be used alone, or two or more of these may be used in combination.

The fluorine-containing (meth)acrylic-modified organosilicon compound may be produced by reacting the epoxy group of the fluorine-containing epoxy-modified organosilicon compound (A) with the carboxyl group of the unsaturated monocarboxylic acid (B) containing a (meth)acrylic group by a known method. Specific examples of the fluorine-containing (meth)acrylic-modified organosilicon compound include the following compounds:

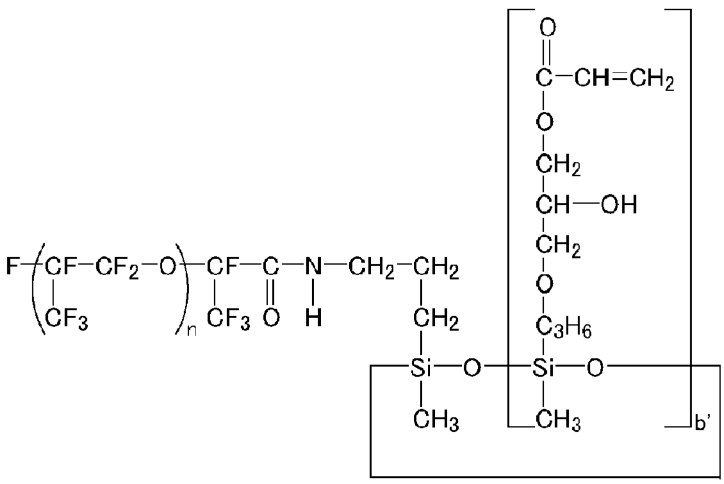

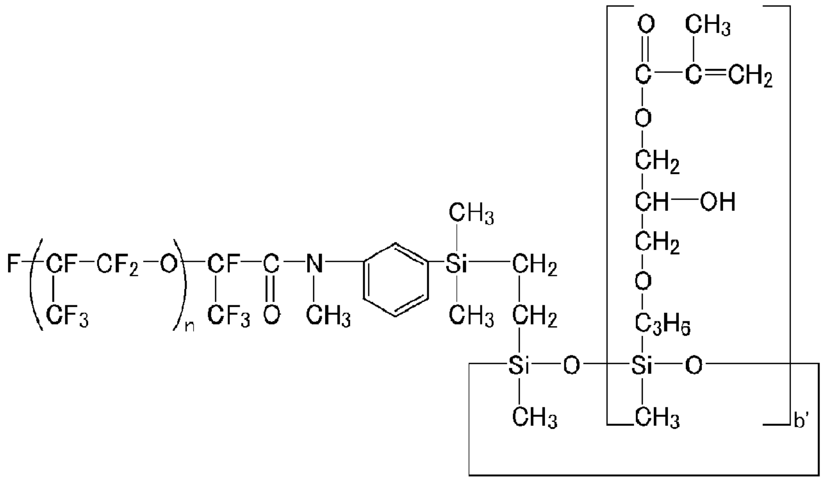

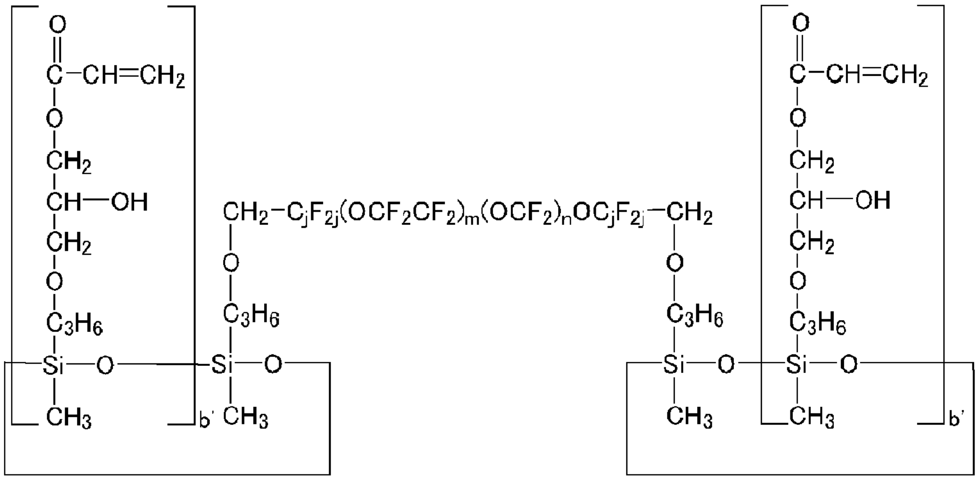

wherein j, m, n, and b' are as defined above.

The fluorine-containing monomer may suitably be a mixture of a fluorine-containing epoxy-modified organosilicon compound as specifically exemplified above and a fluorine-containing (meth)acrylic-modified organosilicon compound as specifically exemplified above. It is particularly preferably a mixture of a fluorine-containing epoxy-modified organosilicon compound and a fluorine-containing (meth)acrylic-modified organosilicon compound as represented by the formulas below:

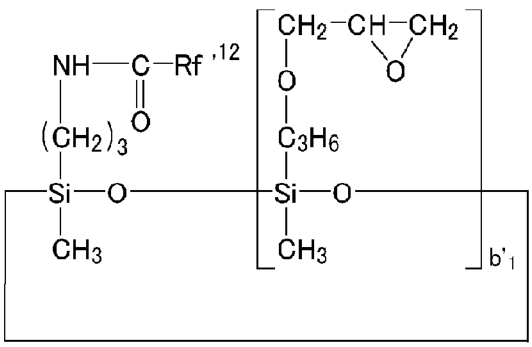

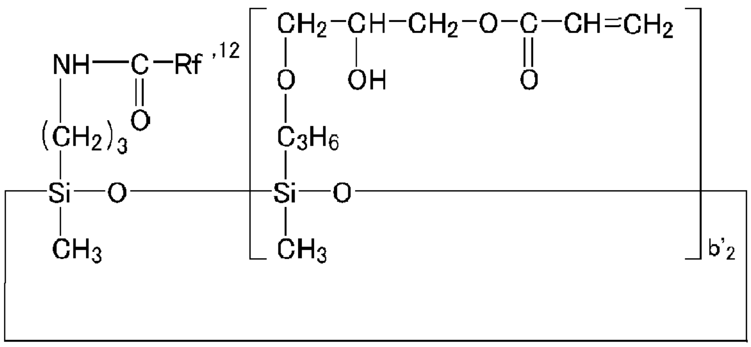

wherein ($b'_1+b'_2$) is 2 to 6.5, and $Rf'^{12}$ is a group represented by the following formula:

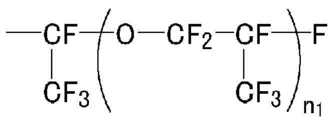

wherein $n_1$ is 2 to 100. In this case, the effects of the present invention can be better achieved.

The fluorine-containing monomer may also be a polyfunctional (meth)acrylate compound containing, per molecule, three or more fluorine atoms and three or more silicon atoms and having a cyclic siloxane represented by the following formula:

$$(Rf^{21}R^{21}SiO)(R^AR^{21}SiO)_h$$

wherein $R^{21}$ represents a hydrogen atom, a methyl group, an ethyl group, a propyl group, or a phenyl group; $Rf^{21}$ represents a fluorine-containing organic group; $R^A$ represents a (meth)acrylic group-containing organic group; and h satisfies $h \geq 2$.

$Rf^{21}$ in the polyfunctional (meth)acrylate compound may be a group represented by $C_tF_{2t+1}(CH_2)_u$- where t represents an integer of 1 to 8, and u represents an integer of 2 to 10, or may be a perfluoropolyether-substituted alkyl group. Specific examples include $CF_3C_2H_4$-, $C_4F_9C_2H_4$-,$C_4F_9C_3H_6$-, $C_8F_{17}C_2H_4$-, $C_8F_{17}C_3H_6$-, $C_3F_7C(CF_3)_2C_3H_6$-, $C_3F_7OC(CF_3)FCF_2OCF_2CF_2C_3H_6$-, $C_3F_7OC(CF_3)FCF_2OC(CF_3)FC_3H_6$-, and $CF_3CF_2CF_2OC(CF_3)FCF_2OC(CF_3)FCONHC_3H_6$-.

Specific examples of $R^A$ include $CH_2$=CHCOO-, $CH_2=C(CH_3)COO$-, $CH_2=CHCOOC_3H_6$-, $CH_2=C(CH_3)COOC_3H_6$-, $CH_2=CHCOOC_2H_4O$-, and $CH_2=C(C_H3)COOC_2H4O$-. Moreover, $R^A$ is preferably bonded to the silicon atom by a Si-O-C bond. The symbol h preferably satisfies $3 \leq h \leq 5$.

The polyfunctional (meth)acrylate compound contains, per molecule, three or more fluorine atoms and three or more silicon atoms, and preferably contains, per molecule, 3 to 17 fluorine atoms and 3 to 8 silicon atoms.

Specific examples of the polyfunctional (meth)acrylate compound include compounds represented by the following formulas.

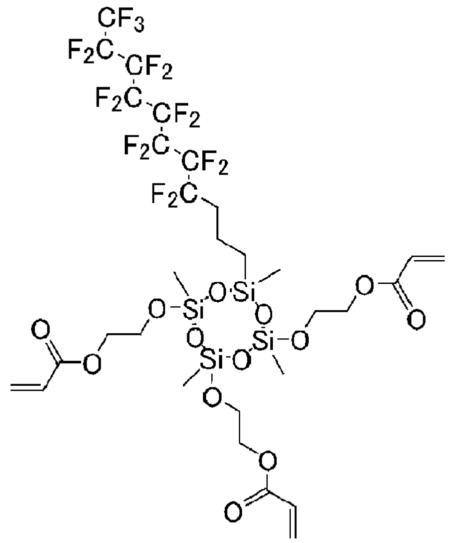

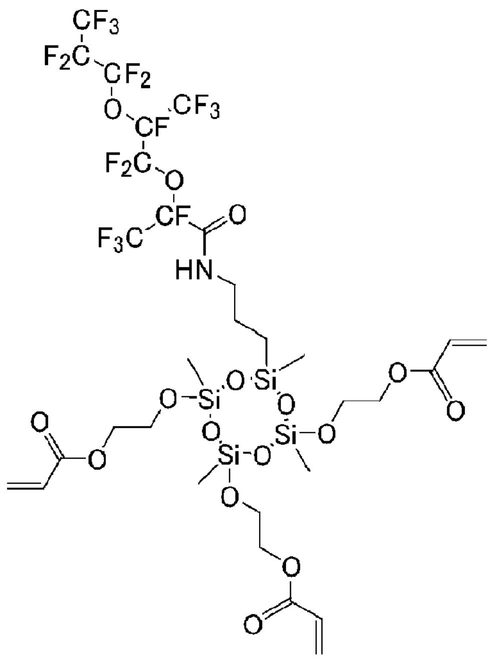

The fluorine-containing monomer is also preferably characterized by an infrared absorption spectrum with absorption peaks at about 1,045 $cm^{-1}$, about 1,180 $cm^{-1}$, about 806 $cm^{-1}$, about 1,720 $cm^{-1}$, about 1,532 $cm^{-1}$, and about 3,350 $cm^{-1}$. In particular, it may suitably be characterized by an infrared absorption spectrum with strong absorption peaks at about 1,045 $cm^{-1}$ and about 1,180 $cm^{-1}$, absorption peaks at about 806 $cm^{-1}$ and about 1,720 $cm^{-1}$, a weak absorption peak at about 1,532 $cm^{-1}$, and a broad weak absorption peak at about 3,350 $cm^{-1}$. Such a monomer can be used to form polymer chains having better properties such as sliding properties.

Moreover, the fluorine-containing monomer is preferably characterized by a $^{13}C$-NMR spectrum in chloroform-d (deuterated chloroform) having signals at chemical shifts of about 13.01, 14.63, 23.04, 40.13, 50.65, 63.54, 68.97, 73.76, 76.74, 77.06, 77.38, 113.21, 114.11, 116.96, 117.72, 118.47, 128.06, 131.38, 156.46, and 166.02 ppm.

The fluorine-containing monomer is also preferably characterized by a $^{1}H$-NMR spectrum in chloroform-d (deuterated chloroform) having signals at chemical shifts of about 3.40, 3.41, 3.49, 3.60, 5.26, 5.58, 6.12, 6.14, 6.40, 6.42, and 6.46 ppm.

In Step 2, the monomer may be radically polymerized as follows. A solution of the monomer or the liquid monomer is applied (sprayed) to the surface of the gasket base material to which a benzophenone or thioxanthone compound or the like is adsorbed or covalently bonded. Alternatively, the gasket base material is immersed in a solution of the monomer or the liquid monomer. Then, the gasket base material is irradiated with light, such as ultraviolet light, to allow the radical polymerization (photoradical polymerization) to proceed, whereby polymer chains can be grown on

the surface of the gasket base material. In another method, after the application, the surface may be covered with a transparent cover of glass, PET, polycarbonate, or other materials, followed by irradiating the covered surface with light, such as ultraviolet light, to allow the radical polymerization (photoradical polymerization) to proceed, whereby polymer chains can be grown on the surface of the gasket base material.

The amount of the radically polymerizable monomer may be selected appropriately depending on, for example, the length of polymer chains to be formed, or the properties to be provided by the chains.

The solvent for application (spraying), the method for application (spraying), the method for immersion, the conditions for irradiation, and other conditions may be conventionally known materials or methods. The solution of the radically polymerizable monomer may be an aqueous solution, or a solution in an organic solvent that does not dissolve the photopolymerization initiator used (e.g. benzophenone or thioxanthone compound). Furthermore, a solution of the radically polymerizable monomer or the liquid radically polymerizable monomer may contain a known polymerization inhibitor such as 4-methylphenol.

The radical polymerization of the monomer is allowed to proceed by light irradiation after the application of a solution of the monomer or the liquid monomer or after the immersion in a solution of the monomer or the liquid monomer. Here, UV light sources with an emission wavelength mainly in the ultraviolet region, such as high-pressure mercury lamps, metal halide lamps, and LED lamps, can be suitably used. The light dose may be appropriately selected in view of polymerization time and uniform reaction progress. In order to prevent inhibition of polymerization due to active gases such as oxygen in the reaction vessel, oxygen is preferably removed from the reaction vessel and the reaction solution during or before the light irradiation. For this purpose, appropriate operations may be performed. For example, an inert gas such as nitrogen gas or argon gas is inserted into the reaction vessel and the reaction solution to discharge active gases such as oxygen from the reaction system and thereby replace the atmosphere in the reaction system with the inert gas. Or the reaction vessel is evacuated to remove oxygen. Also, in order to prevent inhibition of the reaction due to oxygen and other gases, a measure may appropriately be taken; for example, an UV light source is placed such that an air layer (oxygen content: 15% or higher) does not exist between the reaction vessel made of glass, plastic, or other materials and the reaction solution or the gasket base material.

In the case of irradiation with ultraviolet light, the ultraviolet light preferably has a wavelength of 300 to 450 nm, more preferably 300 to 400 nm. Such light allows polymer chains to be formed well on the surface of the gasket base material. The light source may be, for example, a high-pressure mercury lamp, an LED with a center wavelength of 365 nm, or an LED with a center wavelength of 375 nm. In particular, preferred is irradiation with LED light having a wavelength of 300 to 450 nm, more preferably 355 to 380 nm. LEDs or other light sources having a center wavelength of 365 nm, which is close to the excitation wavelength (366 nm) of benzophenone, are particularly preferred in view of efficiency.

The surface modification method I may include (i) Step 3 of extending the polymer chains grown in Step 2 with the same type or a different type of polymer chain, or (ii) Step 3' of attaching a silane compound to the surfaces of the polymer chains grown in Step 2, followed by reaction with a perfluoroether group-containing silane compound to grow functional polymer chains.

Step 3 is not particularly limited as long as it involves further extending the polymer chains. For example, Step 3 may include Step 3-1 of forming polymerization initiation points B on the surfaces of the polymer chains grown in Step 2, and Step 3-2 of radically polymerizing a monomer starting from the polymerization initiation points B to grow polymer chains.

In Step 3-1, the formation of polymerization initiation points B may be carried out by the same techniques as described in Step 1, such as by additionally adsorbing a photopolymerization initiator B onto the surfaces of the formed polymer chains, optionally followed by chemically bonding the photopolymerization initiator B to the surfaces. The photopolymerization initiator B may be as described for the photopolymerization initiator A.

In Step 3-2, a monomer is radically polymerized starting from the polymerization initiation points B to grow polymer chains.

The monomer used in Step 3-2 may be as described for the monomer used in Step 2. In particular, the monomer is preferably (meth)acrylonitrile or a fluorine-containing monomer, more preferably a fluorine-containing monomer, because they provide excellent resistance to liquid leakage and excellent sliding properties.

In Step 3-2, the monomer may be radically polymerized as described for the radical polymerization in Step 2.

In Step 3, the cycle of Steps 3-1 and 3-2 may further be repeated. In this case, the polymer chains that have been chain extended in Steps 3-1 and 3-2 are extended with additional polymer chains.

In Step 3', on the other hand, a silane compound is attached to the surfaces of the polymer chains formed in Step 2, and then reacted with a perfluoroether group-containing silane compound to grow functional polymer chains (functional regions).

The silane compound is not particularly limited, and suitable examples include alkoxysilanes and modified alkoxysilanes. These compounds may be used alone, or two or more of these may be used in combination. Among these, alkoxysilanes are more preferred in order to better achieve the effects of the present invention.

Examples of alkoxysilanes include: monoalkoxysilanes such as trimethylmethoxysilane, triethylethoxysilane,

tripropylpropoxysilane, and tributylbutoxysilane; dialkoxysilanes such as dimethyldimethoxysilane, diethyldiethoxysilane, dipropyldipropoxysilane, and dibutyldibutoxysilane; trialkoxysilanes such as methyltrimethoxysilane, ethyltriethoxysilane, propyltripropoxysilane, and butyltributoxysilane; and tetraalkoxysilanes such as tetramethoxysilane, tetraethoxysilane, tetrapropoxysilane, tetrabutoxysilane, dibutoxydiethoxysilane, butoxytriethoxysilane, and ethoxytriethoxysilane. These compounds may be used alone, or two or more of these may be used in combination. In order to better achieve the effects of the present invention, tetraalkoxysilanes are preferred among these, with tetramethoxysilane, tetraethoxysilane, tetrabutoxysilane, dibutoxydiethoxysilane, butoxytriethoxysilane, and ethoxytributoxysilane being more preferred.

The term "modified alkoxysilane" refers to an alkoxysilane having a substituent such as an amino, carboxyl, hydroxy, or epoxy group, and preferably contains at least one substituent selected from the group consisting of alkyl, amino, carboxyl, hydroxy, and epoxy groups.

In order to better achieve the effects of the present invention, the alkoxysilane or modified alkoxysilane preferably has 4 to 22 carbon atoms, more preferably 4 to 16 carbon atoms.

In order to better achieve the effects of the present invention, the alkoxysilane or modified alkoxysilane preferably contains at least one selected from the group consisting of methoxy, ethoxy, propoxy, and butoxy groups, more preferably ethoxy and/or butoxy group(s), still more preferably ethoxy and butoxy groups.

Examples of commercial products of the silane compound include Primer coat PC-3B (Fluoro Technology, a butoxy/ethoxy tetraalkoxysilane represented by the following formula:

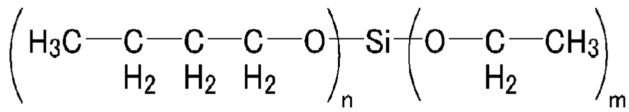

wherein m+n = 4 with n>m>0 on average).

In Step 3', the silane compound may be attached to the surfaces of the polymer chains by any method, and conventionally known methods may appropriately be used, such as bringing the silane compound into contact with the object to be modified on which polymer chains are formed.

The perfluoroether group-containing silane compound may be any silane compound containing a perfluoroether group. For example, it may suitably be a compound represented by the following formula (A) or (B):

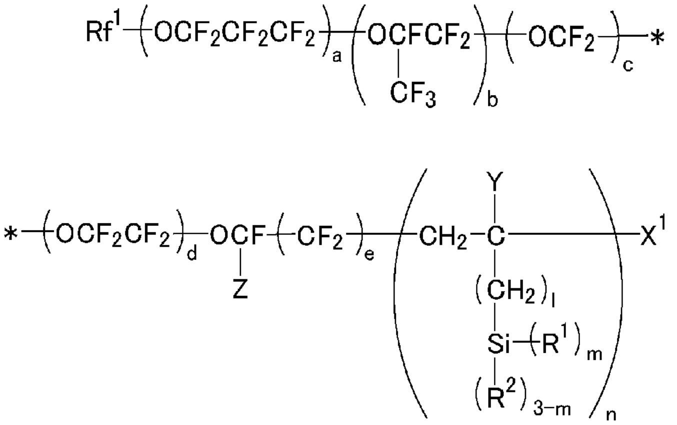

(A)

wherein $Rf^1$ represents a perfluoroalkyl group; Z represents fluorine or a trifluoromethyl group; a, b, c, d, and e are the same as or different from one another and each represent an integer of 0 or 1 or more, provided that (a+b+c+d+e) is 1 or more and the order of the repeating units parenthesized by subscripts a, b, c, d, and e occurring in the formula is not limited to that shown; Y represents hydrogen or a C1-C4 alkyl group; $X^1$ represents hydrogen, bromine, or iodine; $R^1$ represents a hydroxy group or a hydrolyzable substituent such as a C1-C4 alkoxy group; $R^2$ represents hydrogen or a monovalent hydrocarbon group; 1 represents 0, 1, or 2; m represents 1, 2, or 3; and n represents an integer of 1 or more, provided that the two ends marked by * are directly bonded to each other,

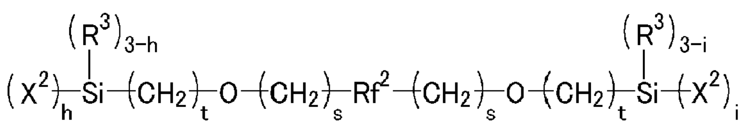

(B)

wherein $Rf^2$ represents a divalent group having a nonbranched linear perfluoropolyalkylene ether structure that contains a unit represented by $-(C_kF_{2k})O-$ where k is an integer of 1 to 6; each $R^3$ is the same or different and represents a C1-C8 monovalent hydrocarbon group; each $X^2$ is the same or different and represents a hydrolyzable group such as a C1-C4 alkoxy group, or a halogen atom; each s is the same or different and represents an integer of 0 to 2; each t is the same or different and represents an integer of 1 to 5; and h and i are the same as or different from each other and each represent 1, 2, or 3.

$Rf^1$ in formula (A) may be any perfluoroalkyl group that can be present in a common organic-containing fluoropolymer, and examples include linear or branched C1-C16 groups. In particular, $CF_3$-, $C_2F_5$-, and $C_3F_7$- are preferred.

In formula (A), each of a, b, c, d, and e represents the number of repeating units in the perfluoropolyether chain which forms the backbone of the fluorine-containing silane compound, and is independently preferably 0 to 200, more preferably 0 to 50. Moreover, (a+b+c+d+e), i.e. the sum of a to e, is preferably 1 to 100. The order of the repeating units parenthesized by subscripts a, b, c, d, and e occurring in formula (A) is not limited to the order shown, and the repeating units may be joined in any order.

Examples of the C1-C4 alkyl group represented by Y in formula (A) include methyl, ethyl, propyl, and butyl groups, and the alkyl group may be linear or branched. When $X^1$ is bromine or iodine, the fluorine-containing silane compound easily forms a chemical bond.

The hydrolyzable substituent represented by $R^1$ in formula (A) is not particularly limited. Preferred examples include halogens, $-OR^4$, $-OCOR^4$, $-OC(R^4)=C(R^5)_2$, $-ON=C(R^4)_2$, and $-ON=CR^6$, where $R^4$ represents an aliphatic hydrocarbon group or an aromatic hydrocarbon group; $R^5$ represents hydrogen or a C1-C4 aliphatic hydrocarbon group; and $R^6$ represents a divalent C3-C6 aliphatic hydrocarbon group. More preferred are chlorine, $-OCH_3$, and $-OC_2H_5$. The monovalent hydrocarbon group represented by $R^2$ is not particularly limited, and preferred examples include methyl, ethyl, propyl, and butyl groups. The hydrocarbon group may be linear or branched.

In formula (A), 1 represents the number of carbon atoms of the alkylene group between the carbon in the perfluoropolyether chain and the silicon attached thereto and is preferably 0; and m represents the number of substituents $R^1$ bonded to the silicon to which $R^2$ is bonded through a bond not attached to $R^1$. The upper limit of n is not particularly critical and is preferably an integer of 1 to 10.

In formula (B), the group represented by $Rf^2$ is preferably, but not limited to, such that when each s is 0, the ends of the $Rf^2$ group bonded to oxygen atoms in formula (B) are not oxygen atoms. Moreover, k in $Rf^2$ is preferably an integer of 1 to 4. Specific examples of the group represented by $Rf^2$ include $-CF_2CF_2O(CF_2CF_2CF_{2O})_jCF_2CF_2-$ in which j represents an integer of 1 or more, preferably an integer of 1 to 50, more preferably 10 to 40; and $-CF_2(OC_2F_4)_p-(OCF_2)_q-$ in which p and q each represent an integer of 1 or more, preferably an integer of 1 to 50, more preferably 10 to 40, and the sum of p and q is an integer of 10 to 100, preferably 20 to 90, more preferably 40 to 80, and the repeating units $(OC_2F_4)$ and $(OCF_2)$ are randomly arranged.

$R^3$ in formula (B) is preferably a C1-C30 monovalent hydrocarbon group, and examples include: alkyl groups such as methyl, ethyl, propyl, butyl, pentyl, hexyl, heptyl, and octyl groups; cycloalkyl groups such as cyclopentyl and cyclohexyl groups; aryl groups such as phenyl, tolyl, and xylyl groups; aralkyl groups such as benzyl and phenethyl groups; and alkenyl groups such as vinyl, allyl, butenyl, pentenyl, and hexenyl groups. Preferred among these is a methyl group.

Examples of the hydrolyzable group represented by $X^2$ in formula (B) include: alkoxy groups such as methoxy, ethoxy, propoxy, and butoxy groups; alkoxyalkoxy groups such as methoxymethoxy, methoxyethoxy, and ethoxyethoxy groups; alkenyloxy groups such as allyloxy and isopropenoxy groups; acyloxy groups such as acetoxy, propionyloxy, butylcarbonyloxy, and benzoyloxy groups; ketoxime groups such as dimethylketoxime, methylethylketoxime, diethylketoxime, cyclopennoxime, and cyclohexanoxime groups; amino groups such as N-methylamino, N-ethylamino, N-propylamino, N-butylamino, N,N-dimethylamino, N,N-diethylamino, and N-cyclohexylamino groups; amide groups such as N-methylacetamide, N-ethylacetamide, and N-methylbenzamide groups; and aminooxy groups such as N,N-dimethylaminooxy and N,N-diethylaminooxy groups. Examples of the halogen atom represented by $X^2$ include chlorine, bromine, and iodine atoms. Preferred among these are a methoxy group, an ethoxy group, an isopropenoxy group, and a chlorine atom.

In formula (B), s is preferably 1, and t is preferably 3. In view of hydrolyzability, h and i are each preferably 3.

For durable mold-releasing effect, the perfluoroether group-containing silane compound preferably has an average molecular weight in the range of 1,000 to 10,000. The average molecular weight can be determined by gel permeation chromatography (GPC) calibrated with polystyrene standards.

Examples of commercial products of the perfluoroether group-containing silane compound include OPTOOL DSX and OPTOOL DSX-E (Daikin Industries, Ltd.), KY-108 and KY-164 (Shin-Etsu Chemical Co., Ltd.), Fluorolink S10 (Solvay Specialty Polymers Japan K.K.), Novec 2702 and Novec 1720 (3M Japan Limited), and FLUOROSURF series such as FLUOROSURF FG-5080SH (Fluoro Technology).

In Step 3', after the attachment of the silane compound, the reaction with the perfluoroether group-containing silane compound may be carried out by any method, and conventionally known methods may appropriately be used,

such as bringing a solution of the perfluoroether group-containing silane compound into contact with the object to be modified to which the silane compound is attached. The solution of the perfluoroether group-containing silane compound may be prepared by using a known solvent that can dissolve the compound (e.g. water, perfluorohexane, acidic water, methanol, ethanol, a mixture of water and methanol or ethanol, or $C_4F_9OC_2H_5$), followed by appropriately adjusting the concentration. The contact between the solution and the object may be made by any method that brings them into contact with each other, such as application, spraying, or immersion.

In the reaction with the perfluoroether group-containing silane compound, the contact (e.g. immersion) is preferably followed by holding at a humidity of 50% or higher. This promotes the reaction so that the effects of the present invention can be well achieved. The humidity is more preferably 60% or higher, still more preferably 80% or higher. The upper limit of the humidity is not particularly critical but is preferably, for example, 100% or lower. The holding time and temperature may be appropriately selected and are preferably, for example, 0.5 to 60 hours and 20°C to 110°C, respectively.

The gasket of the present invention may also be produced by immobilizing polymer chains using a surface modification method II that includes Step I of radically polymerizing a monomer in the presence of a photopolymerization initiator A on the surface of a gasket base material to grow polymer chains.

For example, Step I may be carried out by bringing a photopolymerization initiator A and a monomer into contact with the surface of a gasket base material, followed by irradiation with LED light having a wavelength of 300 to 450 nm to form polymerization initiation points A from the photopolymerization initiator A while radically polymerizing the monomer starting from the polymerization initiation points A to grow polymer chains.

The surface modification method including Step I may include (i) Step II of extending the polymer chains grown in Step I with the same type or a different type of polymer chain; or (ii) Step II' of attaching a silane compound to the surfaces of the polymer chains grown in Step I, followed by reaction with a perfluoroether group-containing silane compound to grow functional polymer chains.

Step II may be carried out by bringing a photopolymerization initiator B and a monomer into contact with the surfaces of the polymer chains formed in Step I, followed by irradiation with LED light having a wavelength of 300 to 450 nm to form polymerization initiation points B from the photopolymerization initiator B while radically polymerizing the monomer starting from the polymerization initiation points B to grow polymer chains. The extension process may be repeated in Step II. In this case, the polymer chains that have been chain extended are further extended.

In Steps I and II, the monomers may be radically polymerized as follows. A solution of the monomer or the liquid monomer, which contains the photopolymerization initiator A or B (e.g. benzophenone or thioxanthone compound), is applied (sprayed) to the surface of the gasket base material or the gasket base material on which polymer chains are formed in Step I. Alternatively, the gasket base material or the gasket base material on which polymer chains are formed in Step I is immersed in a solution of the monomer or the liquid monomer, which contains the photopolymerization initiator A or B. Then, the gasket base material is irradiated with light, such as ultraviolet light, to allow the radical polymerization (photoradical polymerization) to proceed, whereby polymer chains can be grown or extended on the surface of the gasket base material. In another method, for example, the surface may be covered with a transparent cover of glass, PET, polycarbonate, or other materials, followed by irradiating the covered surface with light such as ultraviolet light, as described above. Here, a reducing agent or an antioxidant may be added. The solvent for application (spraying), the method for application (spraying), the method for immersion, the conditions for irradiation, and other conditions may be materials or methods as described above.

In Step II', on the other hand, a silane compound is attached to the surfaces of the polymer chains grown in Step I, and then reacted with a perfluoroether group-containing silane compound to grow functional polymer chains (functional regions). Step II' may be carried out as described in Step 3'.

The polymer chains finally formed by the surface modification method preferably have a degree of polymerization of 500 to 50,000, more preferably 1,000 to 25,000.

The total length of the finally formed polymer chain is preferably 500 to 5,000 nm, more preferably 700 to 2,500 nm. If the total length is shorter than 500 nm, good sliding properties tend not to be obtained. If the total length is longer than 5,000 nm, a further improvement in sliding properties cannot be expected while the cost of raw materials tends to increase due to the use of the expensive monomer. In addition, surface patterns generated by the surface treatment tend to be visible to the naked eyes, thereby spoiling the appearance or deteriorating sealing properties.

In the above-described polymerization processes, two or more types of monomers may simultaneously be radically polymerized starting from the polymerization initiation points A or B. Moreover, multiple types of polymer chains may be grown on the surface of the gasket base material. In the surface modification method, the polymer chains may be crosslinked to one another. In this case, the polymer chains may be crosslinked to one another by ionic crosslinking, crosslinking by a hydrophilic group containing an oxygen atom, or crosslinking by a halogen group such as iodine. Crosslinking by UV irradiation or electron beam irradiation may also be employed.

The surface of the gasket base material may be at least partially or entirely provided with polymer chains. In particular, in view of sliding properties and other properties, preferably at least the sliding surface of the gasket base

material is modified.

EXAMPLES

[Preparation of gasket base material]

Gasket base materials (isoprene unit-containing chlorobutyl rubber with a degree of unsaturation of 1% to 2%) having the shape (Fig. 1, three annular projections (first projection, intermediate projection, and bottom projection)) and surface roughnesses Ra indicated in Table 1 were prepared by crosslinking by triazine (vulcanization at 180°C for 10 minutes) using molds having the respective surface roughnesses. The surface roughness Ra of the surface of each gasket base material was controlled by appropriately varying the surface roughness of the mold (or varying the particle size of the abrasive used in the final finishing step in the production of the mold).

(Example 1)

The gasket base material indicated in Table 1 was immersed in a 3 wt% solution of benzophenone in acetone for 5 minutes so that benzophenone was adsorbed onto the surface of the gasket base material, followed by drying.

The dried gasket base material was immersed in a 2.5 M acrylamide aqueous solution in a glass reaction vessel and subsequently irradiated with LED-UV light having a wavelength of 365 nm for 200 minutes to cause radical polymerization, whereby polymer chains were grown on the rubber surface. Accordingly, a desired gasket (Fig. 2) was prepared.

(Example 2)

The gasket base material indicated in Table 1 was immersed in a 3 wt% solution of benzophenone in acetone for 5 minutes so that benzophenone was adsorbed onto the surface of the gasket base material, followed by drying.

The dried gasket base material was immersed in a 2.5 M acrylamide aqueous solution in a glass reaction vessel and subsequently irradiated with LED-UV light having a wavelength of 365 nm for 150 minutes to cause radical polymerization, whereby polymer chains were grown on the rubber surface. Accordingly, a desired gasket (Fig. 2) was prepared.

(Example 3)

The gasket base material indicated in Table 1 was immersed in a 3 wt% solution of benzophenone in acetone so that benzophenone was adsorbed onto the surface of the gasket base material, followed by drying.

The dried gasket base material was immersed in a 2.5 M aqueous mixture of acrylamide and acrylic acid (acrylamide:acrylic acid = 75:25) in a glass reaction vessel and subsequently irradiated with LED-UV light having a wavelength of 365 nm for 60 minutes to cause radical polymerization, whereby polymer chains were grown on the rubber surface. Accordingly, a desired gasket (Fig. 2) was prepared.

(Example 4)

The gasket base material indicated in Table 1 was immersed in a 3 wt% solution of benzophenone in acetone so that benzophenone was adsorbed onto the surface of the gasket base material, followed by drying.

The dried gasket base material was immersed in a 2.5 M aqueous mixture of acrylamide and acrylic acid (acrylamide:acrylic acid = 75:25) in a glass reaction vessel and subsequently irradiated with LED-UV light having a wavelength of 365 nm for 90 minutes to cause radical polymerization, whereby polymer chains were grown on the rubber surface. Accordingly, a desired gasket (Fig. 2) was prepared.

(Example 5)

The gasket base material indicated in Table 1 was immersed in a 2.5 M aqueous mixture of acrylic acid and acrylamide (25:75) (prepared by dissolving 4.5 g of acrylic acid and 13.4 g of acrylamide in 100 mL of water and then dissolving 2 mg of benzophenone in the solution) in a glass reaction vessel, followed by irradiation with LED-UV light having a wavelength of 365 nm for 120 minutes to cause radical polymerization, whereby polymer chains were grown on the rubber surface. Accordingly, a desired gasket (Fig. 2) was prepared.

(Example 6)

The gasket base material indicated in Table 1 was immersed in a 3 wt% solution of benzophenone in acetone

so that benzophenone was adsorbed onto the surface of the gasket base material, followed by drying.

The dried gasket base material was immersed in a 2.5 M aqueous mixture of acrylamide and acrylic acid (acrylamide:acrylic acid = 75:25) in a glass reaction vessel and subsequently irradiated with LED-UV light having a wavelength of 365 nm for 50 minutes to cause radical polymerization, whereby polymer chains were grown on the rubber surface. Thereafter, the surface was washed with water and dried.

Next, the dried vulcanized rubber gasket was again immersed in a 3 wt% solution of benzophenone in acetone for 5 minutes so that benzophenone was adsorbed onto the surfaces of the polymer chains, followed by drying.

Further, a fluorine-containing monomer liquid (a 20 wt% dilution in ethanol of KY-1203 available from Shin-Etsu Chemical Co., Ltd. (a mixture of a fluorine-containing epoxy-modified organosilicon compound and a fluorine-containing (meth)acrylic-modified organosilicon compound as represented by the formulas below)) was applied to the surface of the dried vulcanized rubber gasket, followed by irradiation with LED-UV light having a wavelength of 365 nm for 10 minutes to cause radical polymerization, whereby the polymer chains were extended. Accordingly, a desired gasket (Fig. 2) was prepared.

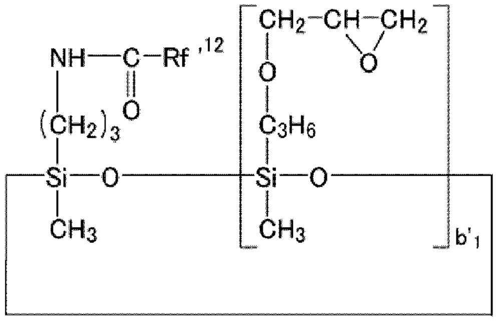

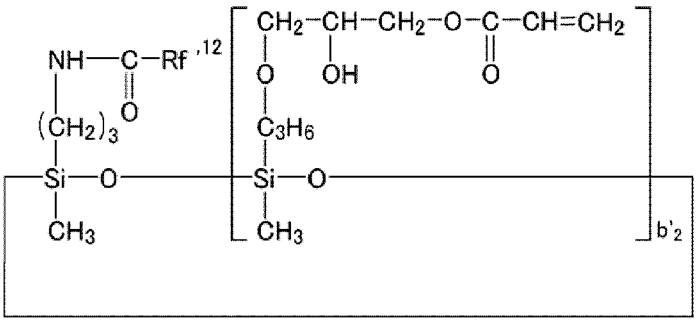

In the formulas, ($b'_1$+$b_{'2}$) is 2 to 6.5, and $Rf'^{12}$ is the following group:

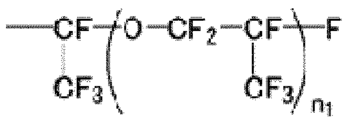

wherein $n_1$ is 2 to 100.

(Example 7)

The gasket base material indicated in Table 1 was immersed in a 3 wt% solution of benzophenone in acetone so that benzophenone was adsorbed onto the surface of the gasket base material, followed by drying.

The dried gasket base material was immersed in a 2.5 M aqueous mixture of acrylamide and acrylic acid (acrylamide:acrylic acid = 75:25) in a glass reaction vessel and subsequently irradiated with LED-UV light having a wavelength of 365 nm for 50 minutes to cause radical polymerization, whereby polymer chains were grown on the rubber surface. Thereafter, the surface was washed with water and dried.

Next, the dried vulcanized rubber gasket was immersed in a silane compound (Primer coat PC-3B available from Fluoro Technology, a butoxy/ethoxy tetraalkoxysilane represented by the above formula), taken out, and dried.

Then, the dried vulcanized rubber gasket was immersed in a 2% solution of a perfluoroether group-containing

silane compound (OPTOOL DSX-E available from Daikin Industries, Ltd., a compound of formula (A)) in $C_4F_9OC_2H_5$ (Novec HFE-7200 available from 3M) and taken out of the solution. The resulting gasket was left at a humidity of 90% for 24 hours to cause a reaction. Thereafter, the gasket was washed with acetone and dried. Accordingly, a desired gasket (Fig. 2) was prepared.

(Example 8)

The gasket base material indicated in Table 1 was immersed in a 3 wt% solution of benzophenone in acetone so that benzophenone was adsorbed onto the surface of the gasket base material, followed by drying.
The dried gasket base material was immersed in a 2.5 M aqueous mixture of acrylamide and acrylic acid (acrylamide:acrylic acid = 75:25) in a glass reaction vessel and subsequently irradiated with LED-UV light having a wavelength of 365 nm for 60 minutes to cause radical polymerization, whereby polymer chains were grown on the rubber surface. Thereafter, the surface was washed with water and dried.
Next, the dried vulcanized rubber gasket was immersed in a silane compound (Primer coat PC-3B available from Fluoro Technology, a butoxy/ethoxy tetraalkoxysilane represented by the above formula), taken out, and dried.
Then, the dried vulcanized rubber gasket was immersed in a 2% solution of a perfluoroether group-containing silane compound (OPTOOL DSX-E available from Daikin Industries, Ltd., a compound of formula (A)) in $C_4F_9OC_2H_5$ (Novec HFE-7200 available from 3M) and taken out of the solution. The resulting gasket was left at a humidity of 90% for 24 hours to cause a reaction. Thereafter, the gasket was washed with acetone and dried. Accordingly, a desired gasket (Fig. 2) was prepared.

(Example 9)

The gasket base material indicated in Table 1 was immersed in a 3 wt% solution of benzophenone in acetone so that benzophenone was adsorbed onto the surface of the gasket base material, followed by drying.
The dried gasket base material was immersed in a 2.5 M aqueous mixture of acrylamide and acrylic acid (acrylamide:acrylic acid = 75:25) in a glass reaction vessel and subsequently irradiated with LED-UV light having a wavelength of 365 nm for 75 minutes to cause radical polymerization, whereby polymer chains were grown on the rubber surface. Thereafter, the surface was washed with water and dried.
Next, the dried vulcanized rubber gasket was immersed in a silane compound (Primer coat PC-3B available from Fluoro Technology, a butoxy/ethoxy tetraalkoxysilane represented by the above formula), taken out, and dried.
Then, the dried vulcanized rubber gasket was immersed in a 2% solution of a perfluoroether group-containing silane compound (OPTOOL DSX-E available from Daikin Industries, Ltd., a compound of formula (A)) in $C_4F_9OC_2H_5$ (Novec HFE-7200 available from 3M) and taken out of the solution. The resulting gasket was left at a humidity of 90% for 24 hours to cause a reaction. Thereafter, the gasket was washed with acetone and dried. Accordingly, a desired gasket (Fig. 2) was prepared.

(Example 10)

A gasket (Fig. 2) was prepared as in Example 7, except that the gasket base material indicated in Table 1 was used.

(Comparative Example 1)

The gasket base material indicated in Table 1 was used as it was.

(Comparative Example 2)

The gasket base material indicated in Table 1 was used as it was.

(Comparative Example 3)

The gasket base material indicated in Table 1 was immersed in a 3 wt% solution of benzophenone in acetone so that benzophenone was adsorbed onto the surface of the gasket base material, followed by drying.
The dried gasket base material was immersed in a 2.5 M acrylamide aqueous solution in a glass reaction vessel and subsequently irradiated with LED-UV light having a wavelength of 365 nm for 240 minutes to cause radical polymerization, whereby polymer chains were grown on the rubber surface. Accordingly, a desired gasket was prepared.

(Comparative Example 4)

The gasket base material indicated in Table 1 was immersed in a 3 wt% solution of benzophenone in acetone for 5 minutes so that benzophenone was adsorbed onto the surface of the gasket base material, followed by drying.
The dried gasket base material was immersed in a 2.5 M acrylamide aqueous solution in a glass reaction vessel and subsequently irradiated with LED-UV light having a wavelength of 365 nm for 200 minutes to cause radical polymerization, whereby polymer chains were grown on the rubber surface. Accordingly, a desired gasket was prepared.
The gaskets prepared in the examples and comparative examples were evaluated as follows.

(Surface roughness Ra)

The surface roughness was measured contactless at four points (on the first peak) for each of the gasket base materials and gaskets using a laser microscope. The average of the four Ra values was determined as the surface roughness Ra (the average of the center-line surface roughnesses Ra defined in JIS B0601-2001).

(Polymer chain length)

To determine the length of the polymer chain formed on the surface of each gasket, a cross-section of the gasket with polymer chains formed thereon was analyzed using an SEM at an accelerating voltage of 15 kV and a magnification of 1,000 times. The thickness of the polymer layer photographed was taken as the polymer chain length.

(Sliding properties (Friction resistance))

To determine the friction resistance of the surface of each gasket, the gasket prepared in each of the examples and comparative examples was inserted into a COP resin barrel of a syringe and then pushed towards the end of the barrel (push rate: 30 mm/min) using a tensile tester while friction resistance was measured. The friction resistance of each example is expressed as a friction resistance index using the equation below, with Comparative Example 1 set equal to 100. A lower index indicates a lower friction resistance.

(Friction resistance index) = (Friction resistance of each example)/(Friction resistance of Comparative Example 1) × 100

(Resistance to liquid leakage)

The gasket prepared in each of the examples and comparative examples was inserted into a COP resin barrel of a syringe. A solution of red food coloring in water was introduced into the barrel, and the barrel was sealed with a cap. After storage at 40°C for two weeks, one month, three months, and six months, the barrel was visually observed for liquid leakage.

[Table 1]

| | Surface roughness Ra (first projection of gasket base material before immobilization of polymer chains) | Surface roughness Ra (first projection of gasket after immobilization of polymer chains) | Monomer used in first layer | Monomer used in second layer | Polymer chain length (nm) | Sliding properties | Resistance to liquid leakage |
|---|---|---|---|---|---|---|---|
| Example 1 | 0.48 | 0.95 | Acrylamide | None | 5000 | 2.4 | Pass |
| Example 2 | 0.48 | 0.78 | Acrylamide | None | 5000 | 2.3 | Pass |
| Example 3 | 0.48 | 0.77 | Acrylamide/ Acrylic acid | None | 4200 | 2.5 | Pass |
| Example 4 | 0.48 | 0.84 | Acrylamide/ Acrylic acid | None | 4600 | 2.6 | Pass |
| Example 5 | 0.48 | 0.69 | Acrylamide/ Acrylic acid | None | 3800 | 2.9 | Pass |
| Example 6 | 0.48 | 0.74 | Acrylamide/ Acrylic acid | KY-1203 | 2500 | 1.6 | Pass |
| Example 7 | 0.82 | 0.57 | Acrylamide/ Acrylic acid | Primer coat+DSX-E | 1000 | 1.8 | Pass |
| Example 8 | 0.82 | 0.77 | Acrylamide/ Acrylic acid | Primer coat+DSX-E | 1200 | 2.1 | Pass |
| Example 9 | 0.82 | 0.94 | Acrylamide/ Acrylic acid | Primer coat+DSX-E | 1400 | 1.9 | Pass |
| Example 10 | 0.48 | 0.55 | Acrylamide/ Acrylic acid | Primer coat+DSX-E | 1100 | 1.8 | Pass |
| Comparative Example 1 | 0.82 | - | No graft polymer | None | 0 | 100 | Pass |
| Comparative Example 2 | 1.23 | - | No graft polymer | None | 0 | 100 | Some leakage after six-month storage at 40°C |
| Comparative Example 3 | 0.82 | 1.37 | Acrylamide | None | 7300 | 2.8 | Some leakage after three-month storage at 40°C |

(continued)

| | Surface roughness Ra (first projection of gasket base material before immobilization of polymer chains) | Surface roughness Ra (first projection of gasket after immobilization of polymer chains) | Monomer used in first layer | Monomer used in second layer | Polymer chain length (nm) | Sliding properties | Resistance to liquid leakage |
|---|---|---|---|---|---|---|---|
| Comparative Example 4 | 0.82 | 1.14 | Acrylamide | None | 6000 | 3.2 | Some leakage after six-month storage at 40°C |

Table 1 shows that the surfaces of the gaskets of the examples exhibited greatly reduced friction resistance and thus had good sliding properties. Moreover, the gaskets having a predetermined surface roughness or less also presented no particular problem with liquid leakage. In contrast, the gaskets of Comparative Examples 1 and 2 exhibited high resistance to sliding upon insertion into the barrel, and also had very poor sliding properties. The gasket (after the immobilization of polymer chains) of Comparative Example 3 had a high surface roughness and exhibited some liquid leakage after three-month storage. The gasket (after the immobilization of polymer chains) of Comparative Example 4 had a slightly high surface roughness and exhibited some liquid leakage after six-month storage.

These results demonstrate that the gaskets in which polymer chains were immobilized on the sliding portion, and further in which the first projection and other projections had a low surface roughness achieved a balanced improvement in sliding properties and resistance to liquid leakage.

Thus, the gasket of the present invention, when used as a gasket of a syringe plunger, provides sufficient resistance to liquid leakage while reducing the friction of the plunger against the syringe barrel, and therefore enables an easy and accurate treatment with the syringe. Moreover, the gasket has a small difference between static and kinetic coefficients of friction, and therefore it allows the start of pushing the plunger and the subsequent inward movement of the plunger to be smoothly carried out without pulsation. Further, by using a syringe barrel made of a thermoplastic elastomer in which polymer chains are formed on the inner surface, the treatment with the syringe can also be facilitated as described above.

REFERENCE SIGNS LIST

1: gasket base material (before immobilization of polymer chains)
2: gasket (after immobilization of polymer chains)
12: top surface
13: bottom surface
14: sliding portion (cylindrical portion)
14a: first projection
14b: intermediate projection
14c: bottom projection
21: polymer chain

## Claims

**1.** A gasket, comprising a gasket base material whose surface is at least partially provided with immobilized polymer chains,
the gasket having a sliding surface provided with multiple annular projections,
the annular projections including a first projection nearest to a top surface of the gasket,
the first projection having a surface roughness Ra of 1.0 or less.

**2.** The gasket according to claim 1,
wherein the first projection has a surface roughness Ra of 0.8 or less.

**3.** The gasket according to claim 1,
wherein the first projection has a surface roughness Ra of 0.6 or less.

**4.** The gasket according to any one of claims 1 to 3,
wherein the gasket base material has a surface roughness Ra of 1.0 or less.

**5.** The gasket according to any one of claims 1 to 3,
wherein the gasket base material has a surface roughness Ra of 0.8 or less.

**6.** The gasket according to any one of claims 1 to 3,
wherein the gasket base material has a surface roughness Ra of 0.6 or less.

**7.** The gasket according to any one of claims 1 to 6,
wherein the polymer chains are immobilized by a surface modification method I comprising:

Step 1 of forming polymerization initiation points A on the surface of the gasket base material; and
Step 2 of radically polymerizing a monomer starting from the polymerization initiation points A to grow polymer chains.

**8.** The gasket according to claim 7, wherein the surface modification method I comprises:

Step 3 of extending the polymer chains grown in Step 2 with the same type or a different type of polymer chain; or
Step 3' of attaching a silane compound to surfaces of the polymer chains grown in Step 2, followed by reaction with a perfluoroether group-containing silane compound to grow functional polymer chains.

**9.** The gasket according to claim 7 or 8, wherein Step 1 comprises adsorbing a photopolymerization initiator A onto the surface of the gasket base material, optionally followed by irradiation with LED light having a wavelength of 300 to 450 nm, to form polymerization initiation points A from the photopolymerization initiator A on the surface.

**10.** The gasket according to any one of claims 7 to 9, wherein Step 2 comprises radically polymerizing a monomer starting from the polymerization initiation points A by irradiation with LED light having a wavelength of 300 to 450 nm to grow polymer chains.

**11.** The gasket according to any one of claims 1 to 6, wherein the polymer chains are immobilized by a surface modification method II comprising
Step I of radically polymerizing a monomer in the presence of a photopolymerization initiator A on the surface of the gasket base material to grow polymer chains.

**12.** The gasket according to claim 11, wherein the surface modification method II comprises:

Step II of extending the polymer chains grown in Step I with the same type or a different type of polymer chain; or
Step II' of attaching a silane compound to surfaces of the polymer chains grown in Step I, followed by reaction with a perfluoroether group-containing silane compound to grow functional polymer chains.

**13.** The gasket according to claim 11 or 12, wherein Step I comprises radically polymerizing a monomer by irradiation with LED light having a wavelength of 300 to 450 nm to grow polymer chains.

**14.** The gasket according to any one of claims 1 to 13, wherein the polymer chains have a length of 500 to 5,000 nm.

Fig. 1

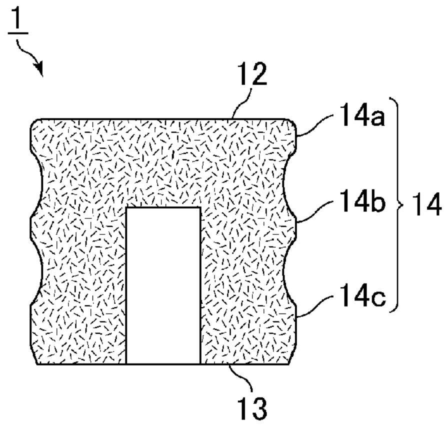


Fig. 2

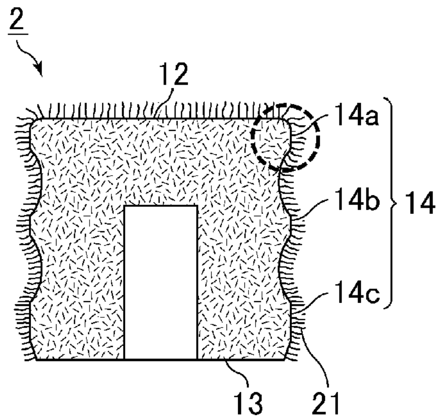


Fig. 3

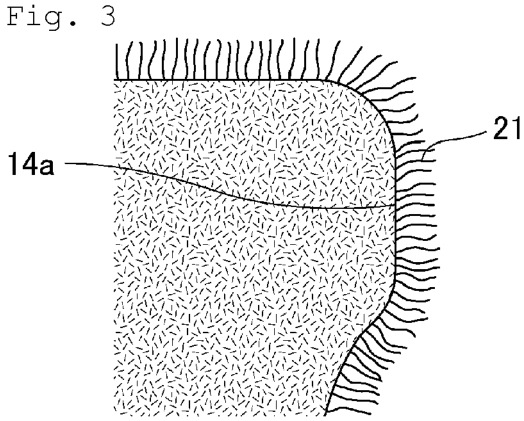

Europäisches Patentamt
European Patent Office
Office européen des brevets

# EUROPEAN SEARCH REPORT

Application Number
EP 17 20 4605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 980 458 A1 (SUMITOMO RUBBER IND [JP]) 3 February 2016 (2016-02-03) * paragraphs [0092], [0098]; claims 1-15; figures 1-3 * | 1-13 | INV. F16J1/00 A61M5/00 B29C39/00 B32B25/00 F16J15/3204 F16J15/3284 |
| X | WO 2009/030976 A1 (BECTON DICKINSON FRANCE [FR]; BOULANGE LAURENCE [FR]; DOMANGE SEVERINE) 12 March 2009 (2009-03-12) * claims 1-19; figure 2 * | 1-13 | |
| X | WO 2009/030975 A1 (BECTON DICKINSON FRANCE [FR]; BOULANGE LAURENCE [FR]; CROZET FREDERIQU) 12 March 2009 (2009-03-12) * claims 1-13; figure 2 * | 1-13 | |
| X | EP 0 879 611 A2 (DAIKYO SEIKO LTD [JP]) 25 November 1998 (1998-11-25) * claims 1-6; figure 3e * | 1-13 | |
| X | US 2016/101239 A1 (ISHIDA NAOYUKI [JP] ET AL) 14 April 2016 (2016-04-14) * paragraphs [0041], [0051]; claims 1-5; figure 2B * | 1-13 | TECHNICAL FIELDS SEARCHED (IPC) |
| X | EP 2 957 310 A1 (SUMITOMO RUBBER IND [JP]) 23 December 2015 (2015-12-23) * paragraph [0049]; claims 1-6; figure 1 * | 1-13 | F16J A61M B29C B32B |

2

The present search report has been drawn up for all claims

| Place of search | Date of completion of the search | Examiner |
|---|---|---|
| The Hague | 22 May 2018 | Regaud, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 20 4605

This annex lists the patent family members relating to the patent documents cited in the above-mentioned European search report. The members are as contained in the European Patent Office EDP file on
The European Patent Office is in no way liable for these particulars which are merely given for the purpose of information.

22-05-2018

| Patent document cited in search report | Publication date | Patent family member(s) | Publication date |
|---|---|---|---|
| EP 2980458 A1 | 03-02-2016 | EP 2980458 A1<br>JP 2016033401 A<br>US 2016033042 A1 | 03-02-2016<br>10-03-2016<br>04-02-2016 |
| WO 2009030976 A1 | 12-03-2009 | NONE | |
| WO 2009030975 A1 | 12-03-2009 | NONE | |
| EP 0879611 A2 | 25-11-1998 | AT 199650 T<br>CA 2238222 A1<br>DE 69800582 D1<br>DE 69800582 T2<br>DK 0879611 T3<br>EP 0879611 A2<br>JP 3387775 B2<br>JP H10314305 A<br>US 6090081 A | 15-03-2001<br>22-11-1998<br>19-04-2001<br>28-06-2001<br>16-07-2001<br>25-11-1998<br>17-03-2003<br>02-12-1998<br>18-07-2000 |
| US 2016101239 A1 | 14-04-2016 | CN 105500561 A<br>EP 3006069 A1<br>JP 2016077354 A<br>US 2016101239 A1 | 20-04-2016<br>13-04-2016<br>16-05-2016<br>14-04-2016 |
| EP 2957310 A1 | 23-12-2015 | CN 105288796 A<br>EP 2957310 A1<br>JP 6270275 B2<br>JP 2016002355 A<br>KR 20150145171 A<br>US 2015367076 A1 | 03-02-2016<br>23-12-2015<br>31-01-2018<br>12-01-2016<br>29-12-2015<br>24-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004298220 A
- JP 2010142573 A